# EUROPEAN PATENT SPECIFICATION

(11) **EP 4 480 399 B1**
(45) Date of publication and mention of the grant of the patent: **16.09.2026**
(21) Application number: 23194403.4
(22) Date of filing: 30.08.2023
(51) Int. Cl.: A61B 5/01, A61B 5/024

(54) **SYSTEM FOR SELECTIVE ADAPTIVE ILLUMINATION FOR THE NEEDS OF THE OPTICAL DETECTION OF VITAL SIGNS**
SYSTEM ZUR SELEKTIVEN ADAPTIVEN BELEUCHTUNG FÜR DIE BEDÜRFNISSE DER OPTISCHEN ERFASSUNG VON VITALZEICHEN
SYSTÈME D'ÉCLAIRAGE ADAPTATIF SÉLECTIF POUR LES BESOINS DE DÉTECTION OPTIQUE DE SIGNES VITAUX

(30) Priority: 20.06.2023 PL 44527023
(43) Date of publication of application: 25.12.2024
(73) Proprietor: Akademia Gorniczo-Hutnicza im. Stanislawa Staszica w Krakowie, 30-059 Krakow (PL)
(72) Inventor: AUGUSTYNIAK, Piotr, 31-457 Kraków (PL); PRZYBYLO, Jaromir, 32-050 Skawina (PL); MISKOWICZ, Marek, 31-621 Kraków (PL)
(74) Representative: Wlasienko, Jozef

(56) References cited:
- EP-B1- 2 948 059
- WO-A1-2014/087310
- US-A1- 2016 206 216
- US-A1- 2017 095 170

## Description

### FIELD OF THE INVENTION

The invention relates to a system for selective adaptive illumination for the needs of the optical detection of vital signs, in particular for monitoring these signs, especially at home while the person subjected to the examination is sleeping.

The present solution according to the invention generally relates to the field of metrology dealing with the quantitative measurements of radiation energy and physical quantities related therewith, and thus the efficiency of radiation sources and effects of radiation, focusing in particular on the measurements of vital signs for diagnostic purposes, especially measurements of the pulse, heart rhythm, pressure or blood flow with the use of non-contact techniques, in particular recording series of images.

### BACKGROUD OF THE INVENTION

Basically, measurement diagnostic methods can be divided into body contact and non-contact methods. The solution according to the invention focuses on the latter methods, namely on non-contact methods involving registration of a series of images; in other words, reception of a video stream of exposed parts of human body, making it possible to obtain a reliable signal in a non-contact manner.

At this point, it should be explained that the method of recording a series of images is essentially based on using the way in which the skin reflects light, which changes slightly due to the blood circulating in the arteries and veins, with each heartbeat. These changes are not visible with the naked eye but, when captured on a camera and subjected to computer processing, they become evident. When recording images, cameras capture information concerning red, green and blue colour. Information concerning the pulse is provided by green colour. This is because the red blood pigment, haemoglobin, absorbs green light most strongly. When the heart delivers more blood to the arteries under the skin, less green light is reflected because the rest is absorbed. The camera image is less green.

### DESCRIPTION OF THE PRIOR ART

The prior art knows a method of photoplethysmography which is based on the optical measurement of changes in the translucency of tissue (e.g. of a finger or ear lobe) depending of the intensity of blood flow. It is a non-invasive contact method; the energy of light introduced into the tissue has negligible values comparable with natural illumination. To limit the influence of external illumination is in necessary to contact the transmitting and receiving optode with the tissue (e.g. of the examined person's skin). Spectral photoplethysmography makes it possible to examine not only the pulse but also tissue composition. In a simplified version used for detecting the percentage content of oxyhemoglobine, it uses two sources of light of different colours and is known as pulse oximetry. Pulse oximetry is easy to use and common, and provides two basic haemodynamic parameters in a single measurement: the pulse and blood oxygen saturation (SpO2). Pulse oximetry is sensitive to light interference and requires sources and detectors (usually semiconductor detectors) that adhere to the skin.

There is also known a video plethysmography (VPG) method, which was proposed by Verkruysse [1] in 2008 as a non-contact method for measuring the pulse. This method essentially consists in using a standard video camera to observe the face and detect pulsation changes in the skin colour caused by the capillary blood flow. The VPG method is a technique that uses ambient light (also referred to as ambient illumination), and numerous studies indicate that a change in the colour of illuminating light (the Sun, a discharge lamp, an incandescent lamp) is a factor that interferes with or even prevents reliable detection of the pulse. This detection consists in analysing sequences of images of the same section of the face skin and detecting rhythmical changes in one of the components in a selected colour space (RGB, YUV or another) [2-4]. There are known scientific reports on optimizing the transformation of colour space used for detecting the pulse on the basis of a video sequence.

Furthermore, in the prior art there is known a solution by MX Labs company, which has developed Shen.AI technology enabling a remote analysis of cardiovascular parameters, heart rate, blood pressure, heart rate variability (HRV) or respiratory rate. In the case of this technology, an average class smartphone or a suitable application are sufficient. In this solution a camera analyses the composition of light reflected from the skin - remote photoplethysmography - a method that makes it possible to obtain various kinds of signals on the basis of which the pulse and blood pressure are subsequently estimated. This technology visualizes real-time blood flow in the face, enabling non-contact measurement of vital signs (measurement of pulse and its variability) solely by means of a smartphone.

In the prior art, there is known an American patent application No. US20160206216A1, titled: *"Device, system and method for skin detection",* which discloses a device, system and method for skin detection are presented. To enable a reliable, accurate and fast detection the proposed device comprises a thermal sensor input for obtaining thermal sensor data of a scene, a light sensor input for obtaining light sensor data of the scene, and an evaluation unit for analysing the obtained thermal sensor data and the obtained light sensor data and for detecting skin areas within the scene based on said analysis. The mentioned document basically describes a passive multisensor system for vital sign detection based on series of thermal and visual images. The system consists of: long-wave thermal camera and visible light camera. Both devices cooperate to establish measurement area and perform the measurement in order to produce (via software) pulse rate information based on video plethysmographic method. The role of the thermal camera is to detect area within the specific temperature, which may alone or in combination with visible-light information (e.g. skin colour) indicate the region for measurement. It is possible for multiple such regions to be indicated in order to either correlate the pulse information from multiple regions, or select the region, where the information is most robust and reliable.

The mentioned solution in the prior art is a passive method which doesn't use supportive light, robustness of the PPG signal is achieved by adjustment of amplification factors of RGB components collected from the camera and common correction values hold for the whole image, making impossible the individual adjustments for different parts of the body in the same subject. This solution uses thermal camera for detecting the skin (together with visual information) and adjust detection zones in the acquired visual image; consequently this solution is a passive system based on ambient light and not working in total darkness. This system uses multiple measurement zones in order to correlate (by the software) the pulse wave and take a common part as more robust and reliable than either of individual waves.

In the prior art, there is also known an international patent application No. WO2014087310A1, titled: *"Device and method for obtaining vital sign information of a living being",* which discloses a device and method for obtaining vital sign information of a living being. The proposed device comprises a detection unit for receiving light in at least one wavelength interval reflected from at least a region of interest of a living being and for generating an input signal from the received light, a processing unit for processing the input signal and deriving vital sign information of said living being from said input signal by use of remote photoplethysmography, an illumination unit for illuminating at least said region of interest with light, and a control unit for controlling said illumination unit based on said input signal and/or said derived vital sign information. The mentioned document basically describes an active system for obtaining vital sign information of a living being by remote photoplethysmography with use of supportive light. The supportive light is provided by a single source or more illumination elements controlled individually, arranged at different locations and/or with different orientations and/or different light spectrum. Still further, said two or more illumination elements have different parameters, in particular different wavelengths, intensities and/or illumination angles. The system described here assumes the use of more detection elements like cameras to combine the information and select the pulsatility signal from the area with best illumination.

The mentioned solution in the prior art doesn't use thermal camera, but visual light camera to detect and select measurement areas, consequently detection zones may not be tracked in the moving subject in total darkness unless illuminated, and precision of these area shapes depends on illuminating source characteristics. Furthermore this solution uses light sources of various spectral characteristics and the control unit switches them to maintain optimal PPG signal, the optimization seeks for best illumination parameters to yield whole image PPG signal, making impossible adjustments for different parts of the body in the same subject, moreover, it is much less precise in colour, position and shape than beams from the beamer controlled my masks based on thermal image. This solution uses detection elements (cameras) of identical parameters but located at different positions and/or with different orientations, in an embodiment the detection elements are different and/or have different parameters so that the detection element resulting in the best vital sign information can be selected for signal evaluation.

The above-mentioned prior art solutions ignore the possibility of measuring the pulse wave delay and touchless measurements either of blood pressure, or the artery stiffness.

Furthermore, in the prior art, there is also known a European patent application No. EP2948059, titled: *" Device and method for determining a partial carbon dioxide pressure in a subject of interest",* which discloses a device and a related method for determining a blood gas partial pressure in blood in a circulatory system of a subject of interest. A system for ventilating a patient is also disclosed. Temperature-related measurement values indicative of given blood temperature levels are detected. Oxygen saturation measurements of the blood of said subject under consideration of said temperature-related measurement values are derived. A blood gas partial pressure of a monitored subject from said derived oxygen saturation measurements is determined under consideration of present blood temperature levels of said subject. Preferably, pH-representative values attributable to present blood pH-values of the subject are determined. More preferably, the pH-representative values are derived from an oxygen dissociation curve under consideration of changes in said derived oxygen saturation measurements of the blood attributable to detected temperature-related measurement values of the subject's blood.

In the prior art, there is also known an American patent application No. US20170095170A1, titled: *"System and method for obtaining vital sign related information of a living being",* which discloses a device, system and method for obtaining vital sign related information of a living being. The proposed device comprises an input unit for receiving an input signal generated from light received in at least one wavelength interval reflected from a skin region of a living being, said input signal representing vital sign related information from which a vital sign of the living being can be derived, a processing unit for processing the input signal and deriving vital sign related information of said living being from said input signal, an orientation estimation unit for estimating the orientation of said skin region, and a control unit for controlling an illumination unit for illuminating said skin region with light to illuminate said skin region based on the estimated orientation of said skin region and/or for controlling said processing unit to derive vital sign related information from said input signal obtained during time intervals selected based on the estimated orientation of said skin region.

Finally, in the prior art there is known an American patent application no. US20220087550A1, titled: *"Apparatus, method, and non-transitory computer-readable recording medium having stored therein program for blood pressure estimating program",* which discloses an apparatus for estimating a blood pressure with a first pulse wave information detector configured to detect first pulse wave information at a first position of a living body (a person subjected to examination), with a second pulse wave information detector configured to detect second pulse wave information at a second position of the living body (a person subjected to examination), the second position being spaced a distance in a vertical direction from the first position, and a blood pressure estimator configured to estimate, based on pulse wave amplitude information at the first position and the second position. The present solution, however, involves a multi-point (multi-zone) measurement without regard to the shape of zones. The location of zones is determined on the basis of thermal radiation, and the colour of illumination - on the basis of skin colour detection.

The above described prior-art solutions and, more broadly, solutions concerning non-contact measurement diagnostics essentially process data concerning colours and calculate a mean value for visible skin zones - the face, neck and arms. However, one limitation thereof is that they assume an analysis of colour changes in the same fragment of the skin, which results in the need to stabilize e.g. the head, neck, arms or palm of the person being examined. Involuntary movements of body parts, e.g. the head, as well as a change in the illumination, cause fluctuations in the recorded light, regardless of the pulse, which are an interference that reduces the pulse detection reliability. Due to these limitations, no clinical apparatuses (medical products) or household devices (non-medical products) based on video plethysmography (VPG) measurement have been developed, yet.

In view of the above, the present invention is intended to overcome these drawbacks of the prior art, and more precisely, to overcome specific limitations in respect of factors interfering with or even preventing a reliable detection of vital signs, such as the pulse measurement by means of non-contact methods, and more specifically the video plethysmography (VPG) method, due to the skin colour or shade of the person subjected to examination and/or due to variable external illumination at the site of measurement, i.e. the colour and intensity of light.

### SUMMARY OF THE INVENTION

The object of the present invention is to develop a totally novel solution in the form of a system for selective adaptive illumination for the needs of the optical detection of vital signs, in particular for monitoring them.

The proposed system is intended to ensure registration of a series of images of exposed body parts of a person (hereinafter also referred to as an object) subjected to such a measurement, thus making it possible to obtain, in a non-contact way, a reliable measurement signal e.g., in this case of the pulse, independently of the external ambient illumination at the site of measurement, by automatic adaptation of light additionally illuminating the examined person, including in complete darkness, in particular at home while the person subjected to examination is sleeping. Importantly, in the case of the claimed solution the key feature is to adapt the illumination of exposed body parts (e.g. the forehead, cheek, arm, hand, etc.) in terms of the intensity, time, colour, shape and direction of illuminating beams, and ensure that the fields of view/illumination are coincident so that the surfaces being observed and illuminated are coincident and the illumination zones correspond to the exposed body parts of the object i.e. basically, to the height of the entire body of the person subjected to the measurement.

Thus, the solution according to the invention is intended to make it possible to eliminate or significantly reduce the influence of ambient illumination at the site of measurement, i.e. the colour and intensity of illumination, and the colour or shade of the examined person's skin in order to ensure adequate reliability of the measurement of vital signs for diagnostic purposes, in particular to enable measurement of the pulse by VPG method in complete darkness, e.g. while the examined person is sleeping.

The aim of the invention is to integrate electronic sensors of visible light intended for video-plethysmographic (VPG) imagining and thermal radiation used for the detection of exposed body parts of a person subjected to such a measurement, and a surface illuminator used to control illumination into one set which, together with software, will enable simultaneous, independent optimization of the illumination conditions of many selected movable exposed body parts of a person subjected to examination for the purpose of reliable multipoint detection, for example, of the pulse by the video plethysmography (VPG) method.

The essence of the invention is defined by a system for selective adaptive illumination for the needs of the optical detection of vital signs, in particular for monitoring these signs by recording sequences of images of exposed body parts of an object, according to claim 1. Dependent claims 2-6 relate to individual embodiments of the invention according to claim 1.

To achieve the above purposes, according to one aspect of the invention, the invention provides a system for selective adaptive illumination for the needs of the optical detection of vital signs, in particular for monitoring these signs by recording a sequences of images of exposed body parts of an object, comprising: a thermal radiation detector, a surface illuminator and a visible light camera, characterized in that the thermal radiation detector together with the surface illuminator in the form of a programmable computer projector and a full-spectrum visible light camera are combined together to form a measuring head which is connected to a programmable analyser, the measuring head being mounted on a support arm such that the observed and the illuminated surfaces coincide with each other and correspond to the areas of illumination zones of the identified exposed body parts of the object thus making it possible to determine a signal of vital signs by the video plethysmography (VPG) method, through the analysis of brightness variations of the same area in subsequent sequences of images in real time in many simultaneously and independently analysed measurement areas.

Preferably, the thermal radiation detector, the programmable computer projector and the full-spectrum visible light camera are each time equipped with prime lenses with a wide angle of view amounting to at least 45 degrees.

Preferably, the said thermal radiation detector is a thermovision camera with infrared sensitivity in the spectral range of 7.5-14 µm with a relative accuracy of 50 mK, adjusted to register the object's thermal emissivity in the form of a sequence of images with a spatial resolution amounting to at least 2 mm per pixel and temperature resolution amounting to at least 0.5°C, at a thermal image refresh rate amounting to at least 25-30 thermal images per second handled by a separate thread of software for optimization and analysis with the use of the programmable analyser.

Preferably, the said programmable computer projector is equipped with an illuminator adapted for the selective adaptive illumination of the exposed body parts of an object based on the emission of light in the form of illuminating beams corresponding to the number of illumination zones of the identified exposed body parts of the object, wherein each of the illuminating beams is an independent source of light with a separate controlled adjustment of the intensity and colour of illumination, which are variable depending on the conditions at the site of measurement and the object's optical properties, independently for each illuminated area handled by a separate thread of software for optimization and analysis with the use of the programmable analyser.

Preferably, the said full-spectrum visible light camera is adapted for recording light in the visible spectrum, reflected from the illumination zones of the identified exposed body parts of the object in the form of a series of images, wherein the angle of view of the full-spectrum visible light camera coincides with the field of illumination of the programmable computer projector, and the resolution is at least 1024 pixels at an image refresh rate of at least 25-30 images per second handled by a separate thread of software for optimization and analysis with the use of the programmable analyser.

Preferably, the said programmable analyser is equipped with:
- a module for detecting of exposed body parts of the object, adapted to analyse thermal images in real time based on the detection of exceeding a specific threshold value of thermal emissivity relative to the covered parts in order to obtain a set of spatial coordinates determining the location and size of the contours of the illumination zones of the identified exposed body parts of the object to determine a mask of the illuminated areas in order to track them later during their movement;
- a module for detecting of a signal of vital signs adapted to analyse cyclic variations in the brightness of the illumination zones of the identified exposed body parts of the object and to determine subsequent wave values of the examined vital signs, independently for each of the illumination zones of the identified exposed body parts of the object, and
- a module for detecting of the signal quality of the examined vital signs, adapted to compare the energy of the spectrum around the frequencies of the examined vital signs with the total energy of the spectrum and to independently control the intensity and colour of individual illuminating beams generated by the programmable computer projector.

The solution designed according to the aspect proposed above provides the possibility of recording vital signs such as the pulse by the video plethysmography (VPG) method independently of ambient illumination, even in total darkness, making it possible to obtain a reliable measurement signal in a contactless manner, in particular while the person subjected to such a measurement is sleeping, estimating the signal from the video stream.

Moreover, the invention provides the possibility of recording vital signs, such as the pulse, when the monitored person is in motion, while maintaining a constant relative position of the illuminating beam and of the moving exposed part of the body; the measurement is highly precise irrespective of the selected exposed fragment of the skin of any shape and size, even if this part of the body is moving. One unquestionable advantage is that it is possible to use several illuminating beams simultaneously (i.e. a multipoint measurement by the video plethysmography (VPG) method; simultaneous, alternate or asynchronous by means of one camera) and individually automatically select the intensity, position, colour and time for each of them; and the independent handling of individual exposed body parts (illuminating beams and zones for brightness variation analysis) can be performed by concurrent software threads.

Another advantage of the proposed invention is that it is possible to automatically adapt the intensity and colour of the illuminating light to the variable conditions of external illumination and local optical properties of the skin (e.g. complexion) independently for each exposed body part of the person subjected to such a measurement while simultaneously ensuring reliability of the measurement irrespective of the skin colour or shade of the person subjected to such a measurement.

In addition, another advantage of the claimed solution in the form of a system for selective adaptive lighting for the needs of the optical detection of vital signs is the fact this system does not comprise movable elements that would increase its costs and failure frequency, because the speed of response to a change in the examined person's position depends only on the image refresh rate, computer performance and complexity of image analysis algorithms.

### BRIEF DESCRIPTION OF THE FIGURES

The subject matter of the invention is presented in an embodiment, with reference to the attached figures in which:
- **FIG. 1**: shows a schematic diagram of the structure of the system for selective adaptive illumination for the needs of the optical detection of vital signs according to an embodiment of the invention;
- **FIG. 2**: shows a schematic functional diagram of the system for selective adaptive illumination for the needs of the optical detection of vital signs according to the embodiment of the invention;
- **FIG. 3**: shows a graph that illustrates a signal of the brightness values of the red, green and blue component of an RGB image and represents fluctuations in light intensity, which in turn represent the capillary blood pulse, according to the embodiment of the invention;
- **FIG. 4A-C**: shows a graph of the spectral density of a VPG signal, according to the embodiment of the invention;
- **FIG. 5**: shows a graph that illustrates spectra obtained at different light intensities according to the embodiment of the invention;
- **FIG. 6**: shows a graph that illustrates spectra obtained for illumination with light of different shades according to the embodiment of the invention;
- **FIG. 7**: shows a block diagram of optimization which illustrates calibration of the light intensity and colour of an illuminating beam according to the embodiment of the invention;

### DETAILED DESCRIPTION OF THE INVENTION

The subject matter of the invention is described below in detail with reference to the attached figures and embodiments. The invention is defined by the claims.

A person skilled in the art will appreciate that some aspects of the embodiments can be carried out as a system or a software product. Therefore, the embodiments can be in the form of an all-hardware embodiment, all-software embodiment (including firmware, resident software, a microcode, etc.), "circuit", "module" or "system".

Many functional units described in this specification have been marked as modules in order to better highlight their independence in terms of implementation. For example, a module can be implemented as a hardware circuit comprising non-standard circuits or gate arrays, finished semiconductors, such as logic circuits, transistors or other discrete components. A module can also be implemented in programmable hardware devices, such a user-programmable gate arrays, programmable logic circuits, programmable logic devices or the like.

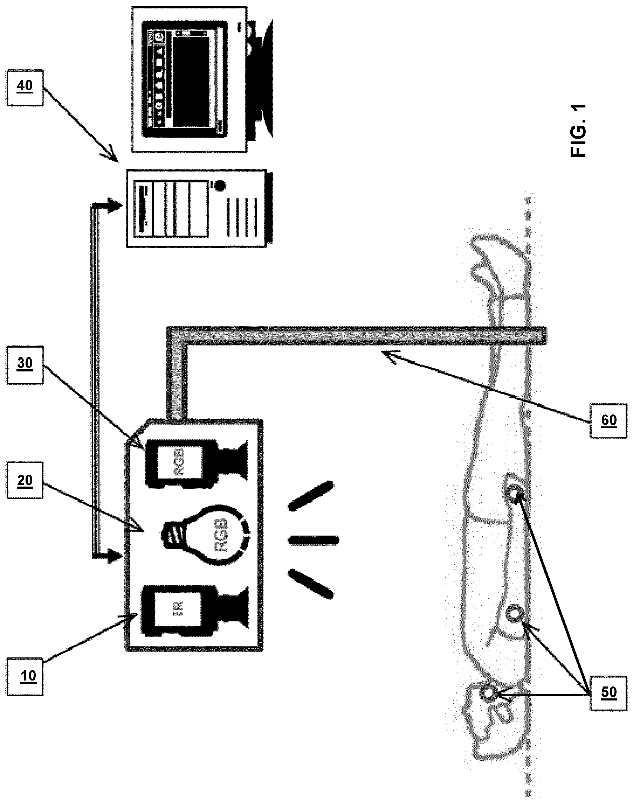

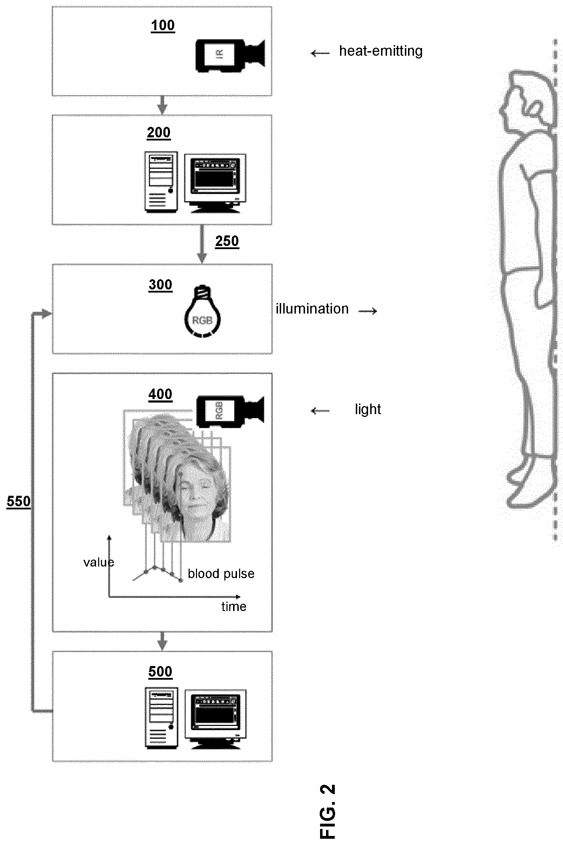

The system for selective adaptive illumination for the needs of the optical detection of vital signs, in this case the pulse, in particular for monitoring the pulse by recording a sequences of images of exposed body parts of a person subjected to examination has been illustrated in the **FIGURES** from **FIG. 1** to **FIG. 2****.**

Basically, the subject system comprises a thermal radiation detector 10 with a programmable computer projector 20 and full-spectrum visible light camera 30 with a common area of view/illumination combined together to form a measuring head which is connected by means of a HDMI digital link with a programmable analyser 40; all connections associated with image transmission use a digital communication format with a capacity considerably exceeding the data stream of the transmitted image. The said measuring head is mounted on a support arm 60 such that the observed and illuminated surfaces coincide with each other and correspond to the areas of illumination zones 50 of the identified exposed body parts of the object thus making it possible to determine the signal of vital signs by the video plethysmography VPG method, through the analysis of brightness variations of the same area in subsequent sequences of images in real time for many simultaneously and independently analysed measurement areas, which method is described in more detail below with reference to an embodiment of the invention.

In the embodiment according to the invention, the thermal radiation detector 10, the programmable computer projector 20 and the visible light camera 30 are each time equipped with prime lenses with a wide angle of view amounting to 45 degrees. This makes it possible to ensure adequate coincidence of the observed and the illuminated surface of given object.

The said thermal radiation detector 10 in the subject solution according to the invention is a thermovision camera with infrared radiation sensitivity amounting in this case to 7.5-14 µm, with a relative accuracy of 50 mK, which is responsible for the detection of exposed body parts in accordance with Step 100, in which the object's thermal emissivity is recorded in the form of the sequences of thermal images having a spatial resolution of 2 mm per pixel, i.e. 1024 pixels per 2 m of the object's height, and a temperature resolution of 0.5°C, at the delivery rate of 30 thermal images per second. At this point, it should be emphasized that a lower rate below 25 thermal images per second does not ensure smooth tracking of the object's movements. It is also worth pointing out that the recorded sequence of thermal images is successively transmitted in a digital form to the module of the exposed body parts detector. This module can be carried out as a separate digital device or in the form of a computer program whose task is to detect exceedance of a specific threshold value of emissivity and process thermal images into a series of masking images subsequently transmitted in a digital form to the programmable computer projector 20.

The said programmable computer projector 20 is equipped with an illuminator adapted for the selective adaptive illumination of the exposed body parts of the object in accordance with Step 300 on the basis of light emission in the form of illuminating beams corresponding to the number of illumination zones 50 of the identified body parts of the object. Each of the illuminating beams is an independent source of light with separately controlled adjustment of intensity and colour of illumination, which are variable depending on the conditions at the site of measurement and the optical features of the object, independently for each illuminated zone handled by a separate thread of software for optimization and analysis with the use of a programmable analyser 40, which is basically responsible for determining the position and shape of the illuminating beams in accordance with Step 200 and controlling illumination geometry in accordance with Step 250.

Essentially, the use of the illuminating beams according to the invention, the image of which can be displayed on the entire body of the object, i.e. the person subjected to examination (i.e. the longer side of the image is greater than the height of the person subjected to examination), provides the following unique advantages for the claimed solution:
- the illuminating beams can be created in any number, simultaneously in several parts of the object, in this case the body of a person subjected to examination. In the solution according to the invention, the number of illuminating beams corresponds to the number of exposed body parts, in this case the illumination zones 50 of identified exposed body parts detected by the thermovision method due to the emission of thermal energy caused by the difference between the temperature of the body and the environment; each of the illuminating beams determines an independent measurement area handled by a separate thread of software for optimization and analysis with the use of the programmable analyser 40;
- the illuminating beams can track the movement of the exposed body parts (e.g. movements of limbs during sleep), the tracking is immediate and noiseless. This is carried out with a speed corresponding to the rate of image reproduction by the programmable computer projector 20, does not require movable elements (only the masking image changes i.e. the location of spots, that is illumination zones 50 of the identified exposed body parts, displayed by the programmable computer projector 20), and thus it is noiseless and non-inertial;
- the illuminating beams can be individually selected in terms of intensity and colour of light to ensure the greatest possible reliability of VPG signal.

The said full-spectrum visible light camera 30 is responsible for the examination, in this case of the pulse, in accordance with Step 400, by recording the visible spectrum of light reflected from the illumination zones 50 of the identified exposed body parts of the object in the form of series of images. Importantly, in the case of the present solution according to the invention the field of view of the full-spectrum visible light camera 30 coincides with the field of illumination of the programmable computer projector 20. The resolution is 1024 pixels, at the image refresh rate amounting in this case to 30 images per second handled by a separate thread of software for optimization and analysis with the use of the programmable analyser 40, which is basically responsible for determining the quality of VPG signal, in accordance with Step 500, and controlling the intensity and colour of illumination, in accordance with Step 550, of the programmable computer projector 20.

Finally, the said system comprises a programmable analyser 40 equipped with three modules: a module for detecting of exposed body parts of the object adapted to analyse thermal images in real time, a module for detecting of the signal of vital signs, in this case it is the pulse determined on the basis of cyclic changes in the brightness of the illumination zones 50 of identified exposed body parts of the object, a module for detecting of the pulse signal quality by comparing the spectrum energy around the frequencies of the examined pulse with the total spectrum energy and using this quality to independently control the intensity and colour of individual illuminating beams.

At this point, it should be underlined that, in order to transfer the qualitative information, the programmable analyser 40 analysing the image from the full-spectrum visible light camera 30 is, in one variant of the embodiment, connected to the programmable analyser 40 analysing thermal images or, in another variant, there are two independently activated threads in the same programmable analyser 40. Moreover, information about the quality of video-plethysmographic signal may be obtained from the signal spectrum (e.g. the Fourier spectrum), in this case of the pulse, obtained from the series of VPG images as: the height of a basic line of its spectrum or the ratio of the pulse signal energy to the noise energy over the entire analysed spectrum. The present solution will be described in more detail in the following part of the description.

### Optimisation of the point of VPG measurement

Details concerning optimization of the point of VPG measurement by comparing spectrum energy and spectrum ranges in which maximums are interpreted as representing the pulse are described below.

At this point, it should be pointed out that, in a standard solution, the VPG method records an image of a specific fragment of the skin, the average colour of which subtly changes with the capillary blood pulse. In areas with thinner epidermis (e.g. the forehead, cheek) these changes are more pronounced, whereas in areas with thicker epidermis (e.g. forearms, lower legs) the changes are less pronounced, due to which the reliability of the pulse determined on the basis of colour changes depends on the site of measurement. Changes associated with the pulse are also more evident on fair skin than on dark skin. Even in the same person, the presence of local tan makes the measurement significantly more difficult. In light of the above, it should be clearly stated that the reliability of measurement of, for example, the pulse determined on the basis of changes in the skin colour depends on the colour space (the definition of colours) in which the image is analysed.

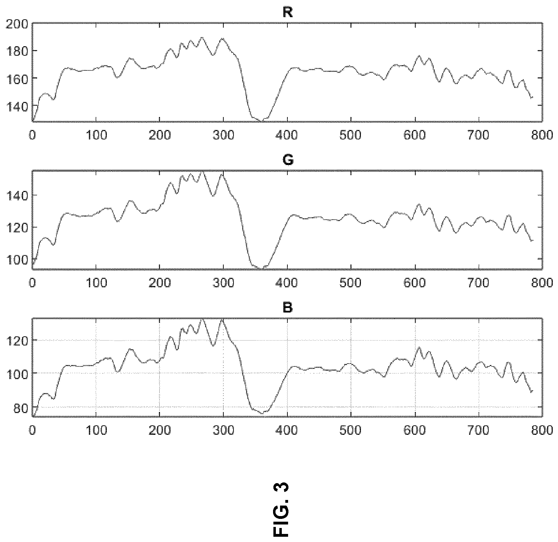

The principle of measurement with the use of video plethysmography VPG according to the invention consists in the analysis of brightness (intensity of reflected light) averaged on a selected fragment of the skin in subsequent images of a video sequence. This sequence is normally recorded in accordance with the accepted video standard of 25 or 30 images per second. In our case, 30 images per second. In the measurement result, the sequence of measured values shows rhythmic fluctuations representing fluctuations in light intensity, which in turn represent the capillary blood pulse, as illustrated in **FIG. 3** wherein the signal is represented by the brightness values of the red R, green G and blue B component thus forming an RGB image - the horizontal axis shows the image numbers; 30 images correspond to 1 s.

The analysis of sequences usually consists in finding the rhythmic component in the range of 50-200 repetitions per minute (i.e. the frequency of 0.83 Hz - 3.33 Hz). The majority of available solutions use a spectral (frequency) analysis for this purpose, e.g. Fourier transformation, in which a sequence *f*(*k*) of values in the time domain is assigned a sequence *F*(*n*) with an identical length K of values in the frequency domain called the spectrum of energy, whose values are coefficients of the expansion of the measurement sequence into a trigonometric sequence: $F \left(n\right) = {\sum }_{k = 0}^{K - 1} \left(f\left(k\right)⋅{e}^{- j \frac{2 πnk}{K}}\right)$ wherein $j = \sqrt{- 1}$ is a versor of an imaginary axis.

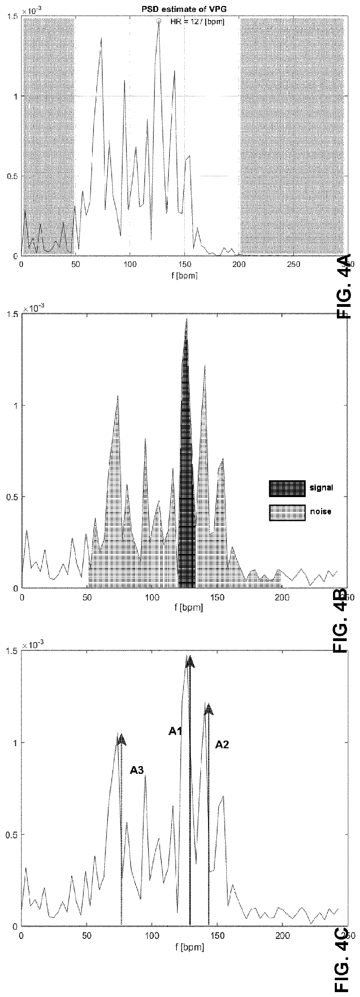

The spectrum determined by the Fourier method is then filtered, and the sequence of its squared values is called the Power Spectrum Density (PSD), which is illustrated in **FIG. 4A-C****,** respectively.

In the spectrum corresponding to the sequence of measured brightness values averaged on the surface of the examined area of the skin, a continuous subset of bins having numbers from N1 to N2, 0 < N1 < N2 < K is determined, such that N1 is the bin number corresponding to the lower frequency of the range i.e. 0.83 Hz, whereas N2 is the bin number corresponding to the upper frequency, i.e. 3.33 Hz, of the range, in which the maximum value is sought. The frequency corresponding to the band with the maximum value is the pulse frequency.

**FIG. 4A** shows an exemplary energy spectrum of a VPG signal, wherein, on the grey background, parts of the spectrum are outside the pulse frequency range. Bin no. 36 has the highest value of energy and corresponds to the frequency of 2.12 Hz, that is 127 beats per minute.

The reliability of the pulse measurement is referred to as Signal-to-Noise Ratio (SNR). It is calculated on the basis of the spectrum as: $SNR = \frac{{P}_{s}}{{P}_{n}}$ wherein: ${P}_{s} = \frac{1}{m 2 - m 1} {\sum }_{m = m 1}^{m 2} F \left(m\right)$ ${P}_{n} = \frac{1}{m 1 - N 1 + N 2 - m 2} \left({\sum }_{m = N 1}^{m 1} F \left(m\right) + {\sum }_{m = m 2}^{N 2} F \left(m\right)\right)$ and m1 and m2 > m1 are numbers of the minimum-value bin that directly precedes (m1) and directly follows (m2) the maximum-value band.

**FIG. 4B** shows an exemplary energy spectrum of a VPG signal wherein the light grey surface represents the sum of the parts of the noise spectrum whereas the dark grey surface represents the sum of the parts of the signal spectrum according to the invention.

Alternatively, the reliability of the pulse measurement can be determined on the basis of spectrum in a simplified way as the ratio of the amplitude of the bin with the highest value A1 to the sum of the amplitudes of two bins with the subsequent highest values A2 and A3, as illustrated in **FIG. 4C** wherein the said three bins with the subsequently highest amplitudes A1, A2 and A3 are marked.

### Adjustment of illumination intensity / colour

A particular advantage of the system according to the invention, resulting from the innovative use of the programmable computer projector 20 as an illuminator, is the possibility of defining illuminating beams as an image displayed by the projector on the body of the object i.e. the person subjected to examination. Each of the illuminating beams is an independent source of light with separate adjustment of intensity and colour.

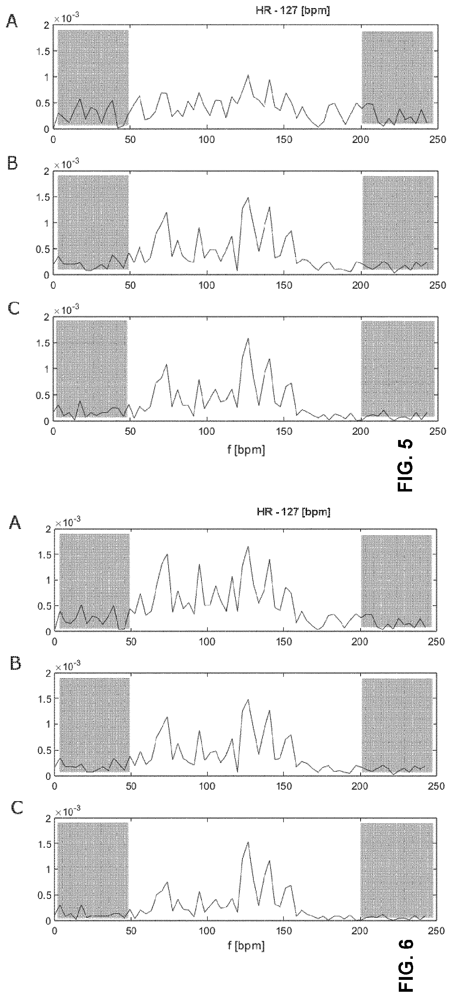

The adjustment of light intensity consists in that, for each illuminating beam, a minimum value of light intensity is selected which ensures intensity of reflected light necessary to achieve sufficient measurement reliability. This minimum value is desirable due to the intrusive nature of the examination (e.g. the possibility of waking up the sleeping person). As the measurements carried out show, up to a certain threshold value, increasing the value of light intensity initially improves the reliability of the VPG measurement, and after exceeding this threshold, no further improvement occurs. The threshold value depends on the colour of light, skin tone and anatomic features (how deep capillary vessels are located), as well as on the sensitivity of the visible light camera. This is shown in **FIG. 5** in the form of a graph illustrating spectra obtained at different light intensities according to the embodiment of the invention wherein A) 5 lx, SNR=0.35, B) 10 lx, SNR=1.2, C) 20 lx, SNR=1.25.

The adjustment of light colour, on the other hand, consists in selecting, for each of the illuminating beams individually, such a value of shade that ensures the highest reflected light component related to the pulsation of blood. This is shown in **FIG. 6** in the form of a graph illustrating spectra obtained at illumination with light of different shades according to the embodiment of the invention wherein in the colour space HSV (Hue, Saturation, Value): A) H=45 (citrine colour), SNR=0.85, B) H=143 (turquoise colour), SNR=1.2, C) H=120 (green colour), SNR=1.45. Apart from the colour of light, the pulsation component depends of the skin tone and anatomic features (how deep the capillary vessels are located). An essential innovation in relation to standard VPG measurements which assume the selection of the optimum colour space (colour definition) of the analysed sequence of images recorded by the camera is the use of light emitted by the programmable computer projector 20 with strictly controlled, easily adjustable colour, which eliminates the influence of the colour space of the full-spectrum visible light camera 30. In addition, the illuminating beams simultaneously illuminating different body parts can take into account local differences in the anatomic factor by the autonomous adjustment of colours for each of them. The adjustment of colours is performed in the colour space HSV after transforming the RGB-HSV image space, and then, after adjusting the shade, the image is transformed again to the RGB space in which the programmable computer projector 20 operates.

### Optimization of light intensity/colour

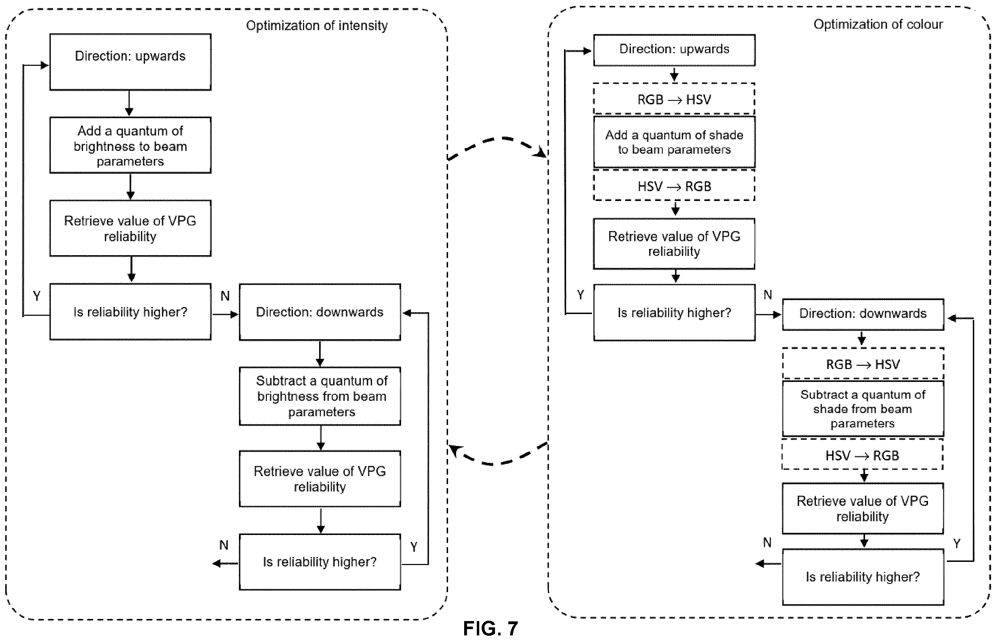

Calibration consists in the alternating adjustment of the light intensity and colour of each illuminating beam individually in order to achieve maximum reliability of the pulse measurement expressed by the SNR value. This is shown in **FIG. 7** in the form of a block diagram according to the embodiment of the invention. Calibration is performed automatically, individually for each illuminating beam in the following cases:
- after identifying, in the thermal image, a new coherent area of an exposed fragment of the skin having an area of at least 100 pixels (which corresponds to about 4 cm²) in order to create a new illuminating beam, if necessary;
- 10 min. after the previous calibration of a given illuminating beam; the database of potential illuminating beams is updated;
- after the reliability of the SNR pulse measurement has dropped below the value of 0.5, if the SNR value obtained as a result of calibration does not exceed 0.5, the illuminating beam is abandoned.

When tracking moving body parts of an object, i.e. the person subjected to examination, the contour and position of the mask change, but the remaining parameters of the associated illuminating beam should remain unchanged. The system according to the invention detects whether the contour detected in a binary thermal image represents the same illuminating beams on subsequent images based on the position of the centre of gravity of the contour. It is determined by the mean value of the pixel coordinates of the mask in the vertical and horizontal axis of the thermal image. If in the successive images the centre of gravity of the subsequent contour is within the previous contour, the contour in the following image is assigned the same label as the contour in the preceding image, as well as the same values of the intensity and colour of illumination. This makes it possible to maintain continuous identification of the beams illuminating exposed body parts moving at a speed of up to 0.6 m/s (the worst case for an illuminating beam having a diameter of 10 pixels and a thermal imaging camera operating with the rate of 30 images per second).

When examining a person's pulse without their awareness (e.g. during sleep) a sharp deterioration in the reliability of VPG may occur or it may be totally impossible to perform the measurement in a specific body part (measurement zone), e.g. it may get covered. Such a situation is immediately detected in the thermal image through the lack of continuity in the identification of the illuminating beam - in the subsequent image there is no contour whose centre of gravity would be within the current contour.

The solution of such a situation is based on the thermal tracking of all exposed (i.e. heat-emitting) body parts and ongoing building the corresponding contours on the basis of thermal image binarization. Then, the system according to the invention, on the basis of size and temperature, automatically indicates (or the operator does it manually) the contours in which calibration of intensity and colour will be performed. The contours that have been indicated are subsequently illuminated and constitute VPG measurement zones. The contours that have not been indicated are continuously tracked in the thermal image as spare ones and may be automatically, or by the operator, added to the list of measurement zones after calibration of intensity and colour has been performed.

The permission to create new measurement zones, the number of active zones, the conditions for changing the hierarchy of zones are the parameters set by the operator before launching the analysing software run by the programmable analyser 40. In particular:
- when measuring the pulse, the VPG signal should ensure maximum reliability; thus, it is possible to create new zones and automatically select, as the main zone, the zone in which reliability is the highest,
- when measuring, for example, the blood pressure, it is desirable to maintain two zones in specific anatomic locations, irrespective of the possible higher reliability in another zone.

The above description of presented embodiments has been provided in order to enable a person skilled in the art to carry out or use the invention. Various modifications of the presented embodiments are also possible, comprising all such changes, modifications and variants that fall within the scope of the appended claims. The basic principles set out herein may therefore be applied in other embodiments without going beyond the scope of the invention. Therefore, the present invention is not intended to be limited to the embodiments presented herein but to accord with the widest scope consistent with the principles and novel features set out herein.

The present solution according to the invention thus offers, with the use of the above mentioned technical means as indicated in the description and Figures, a system for selective adaptive illumination for the needs of the optical detection of vital signs, in particular for the monitoring of those signs by recording sequences of exposed body parts of an object.

### APPLICATION OF THE INVENTION

The invention may be widely applied in many branches such as: healthcare, telemedicine, beauty and wellness, mental, fitness, or InsurTech. In the not too distant future, it could even replace hospital devices for monitoring blood pressure or oxygen levels in the blood.

One particular example of a wide application of this system is non-contact monitoring of the heart rate of a sleeping person with the possibility of detecting common diseases such as sleep apnea or others, whose symptoms during wakefulness are controlled (suppressed) by the somatic nervous system.

### LIST OF REFERENCE NUMBERS

- 10: thermal radiation detector
- 20: programmable computer projector
- 30: full-spectrum visible light camera
- 40: programmable analyser
- 50: illumination zones of identified exposed body parts
- 60: support arm
- 100: detection of exposed body parts
- 200: determining the position and shape of illuminating beams
- 250: controlling of illumination geometry
- 300: selective illumination of exposed body parts
- 400: examining the pulse by VPG method
- 500: determining the quality of VPG signal
- 550: controlling of the intensity and colour of illumination

### LIST OF CITED NON-PATENT LITERATURE

[1] Verkruysse, W., Svaasand, L.O., Nelson, J.S. (2008). Remote plethysmographic imaging using ambient light. Optics express, 16(26), 21434-21445.
[2] Poh, M.Z., McDuff, D.J., Picard, R.W. (2010). Non-contact, automated cardiac pulse measurements using video imaging and blind source separation. Optics express, 18(10), 10762-10774
[3] McDuff, D., Gontarek, S., Picard, R.W. (2014). Remote detection of photoplethysmo-graphic systolic and diastolic peaks using a digital camera. IEEE Transactions on Biomedical Engineering, 61(12), 2948-2954.
[4] Rumiński, J. (2016) Reliability of pulse measurements in videoplethysmography, Metrology and Measurement Systems, Vol. 23, No. 3, pp. 359371.

## Claims

1. A system for selective adaptive illumination for the needs of the optical detection of vital signs, in particular for monitoring these signs by recording a sequence of images of exposed body parts of an object comprising: a thermal radiation detector, a surface illuminator and visible light camera, wherein the thermal radiation detector (10) together with the surface illuminator in the form of a programmable computer projector (20) and the full-spectrum visible light camera (30) are combined together to form a measuring head which is connected to a programmable analyser (40), wherein the measuring head is mounted on the support arm (60) such that the observed and the illuminated surfaces coincide with each other and correspond to the areas of illumination zones (50) of the identified exposed body parts of the object thus making it possible to determine a signal of vital signs by the video plethysmography (VPG) method, through the analysis of brightness variations of the same area in subsequent sequences of images in real time in many simultaneously and independently analysed measurement areas.

2. The system according to claim 1, wherein the thermal radiation detector (10), the programmable computer projector (20) and the full-spectrum visible light camera (30) are each time equipped with prime lenses with a wide angle of view amounting to at least 45 degrees.

3. The system according to claim 1 or 2, wherein the thermal radiation detector (10) is a thermovision camera with infrared sensitivity in the spectral range of 7.5-14 µm with a relative accuracy of 50 mK, adjusted to register the object's thermal emissivity in the form of a sequence of images with a spatial resolution amounting to at least 2 mm per pixel and temperature resolution amounting to at least 0.5°C, at a thermal image refresh rate amounting to at least 25-30 thermal images per second handled by a separate thread of software for optimization and analysis with the use of the programmable analyser (40).

4. The system according to claim 1 or 2, wherein the programmable computer projector (20) is equipped with an illuminator adapted for the selective adaptive illumination of the exposed body parts of an object based on the emission of light in the form of illuminating beams corresponding to the number of illumination zones (50) of the identified exposed body parts of the object, wherein each of the illuminating beams is an independent source of light with a separate controlled adjustment of the intensity and colour of illumination, which are variable depending on the conditions at the site of measurement and the object's optical properties, independently for each illuminated area handled by a separate thread of software for optimization and analysis with the use of the programmable analyser (40).

5. The system according to claim 1 or 2, wherein the full-spectrum visible light camera (30) is adapted for recording light in the visible spectrum, reflected from the illumination zones (50) of the identified exposed body parts of the object in the form of a series of images, wherein the angle of view of the full-spectrum visible light camera (30) coincides with the field of illumination of the programmable computer projector (20), and the resolution is at least 1024 pixels at an image refresh rate of at least 25-30 images per second handled by a separate thread of software for optimization and analysis with the use of the programmable analyser (40).

6. The system according to claim 1 or 3 or 4 or 5, wherein the said programmable analyser (40) is equipped with:
- a module for detecting of exposed body parts of the object, adapted to analyse thermal images in real time based on the detection of exceeding a specific threshold value of thermal emissivity relative to the covered parts of the object in order to obtain a set of spatial coordinates determining the location and size of the contours of the illumination zones (50) of the identified exposed body parts of the object to determine a mask of the illuminated areas in order to track them later during their movement;
- a module for detecting of a signal of vital signs adapted to analyse cyclic variations in the brightness of the illumination zones (50) of the identified exposed body parts of the object and to determine subsequent wave values of the examined vital signs, independently for each of the illumination zones (50) of the identified exposed body parts of the object, and
- a module for detecting of the signal quality of the examined vital signs, adapted to compare the energy of the spectrum around the frequencies of the examined vital signs with the total energy of the spectrum and to independently control the intensity and colour of individual illuminating beams generated by the programmable computer projector (20).

## Patentansprüche

1. System zur adaptiven selektiven Beleuchtung für die optische Erfassung von Vitalparametern, insbesondere zur Überwachung dieser Parameter durch Aufzeichnung von Bildsequenzen freiliegender Körperteile eines Objekts, umfassend: einen Wärmestrahlungsdetektor, eine Flächenbeleuchtungseinrichtung und eine Kamera für sichtbares Licht, **dadurch gekennzeichnet, dass** der Wärmestrahlungsdetektor (10) zusammen mit der Flächenbeleuchtungseinrichtung in Form eines programmierbaren Computerprojektors (20) und einer Vollspektrum-Kamera für sichtbares Licht (30) miteinander verbunden sind und einen Messkopf bilden, der an einen programmierbaren Analysator (40) angeschlossen ist, wobei der Messkopf an einem Tragarm (60) befestigt ist, sodass die beobachteten und beleuchteten Oberflächen übereinstimmen und den Bereichen der Beleuchtungszonen (50) der identifizierten freiliegenden Körperteile des Objekts entsprechen, wodurch es möglich wird, das Signal der Vitalparameter mittels Videoplethysmographie (VPG) zu ermitteln, indem die Helligkeitsschwankungen desselben Bereichs in aufeinanderfolgenden Bildsequenzen in Echtzeit in mehreren gleichzeitig und unabhängig voneinander analysierten Messbereichen ausgewertet werden.

2. System nach Anspruch 1, **dadurch gekennzeichnet, dass** der Wärmestrahlungsdetektor (10), der programmierbare Computerprojektor (20) und die Vollspektrum-Kamera für sichtbares Licht (30) jeweils mit Festbrennweitenobjektiven mit einem Weitwinkel von mindestens 45 Grad ausgestattet sind.

3. System nach Anspruch 1 oder 2, **dadurch gekennzeichnet, dass** es sich bei dem Wärmestrahlungsdetektor (10) um eine Wärmebildkamera mit Infrarot-Empfindlichkeit im Spektralbereich von 7,5-14 µm und einer relativen Genauigkeit von 50 mK handelt, die zur Erfassung der thermischen Emissivität eines Objekts in Form einer Bildsequenz mit einer räumlichen Auflösung von mindestens 2 mm pro Pixel und einer Temperaturauflösung von mindestens 0,5 °C ausgelegt ist, bei einer Wärmebild-Wiederholfrequenz von mindestens 25-30 Wärmebildern pro Sekunde, die von einem separaten Software-Thread zur Optimierung und Auswertung unter Verwendung eines programmierbaren Analysators (40) verarbeitet werden.

4. System nach Anspruch 1 oder 2, **dadurch gekennzeichnet, dass** der programmierbare Computerprojektor (20) mit einer Beleuchtungseinrichtung zur adaptiven selektiven Beleuchtung freiliegender Körperteile des Objekts ausgestattet ist, basierend auf der Emission von Licht in Form von Beleuchtungsstrahlen, die der Anzahl der Beleuchtungszonen (50) der identifizierten freiliegenden Körperteile des Objekts entsprechen, wobei jeder der Beleuchtungsstrahlen eine unabhängige Lichtquelle mit separat steuerbarer Regelung der Beleuchtungsstärke und -farbe darstellt, die je nach den Bedingungen am Messort und den optischen Eigenschaften des Objekts variabel sind, und zwar unabhängig für jeden beleuchteten Bereich, der von einem separaten Software-Thread zur Optimierung und Auswertung unter Verwendung eines programmierbaren Analysators verarbeitet wird (40).

5. System nach Anspruch 1 oder 2, **dadurch gekennzeichnet, dass** die Vollspektrum-Kamera (30) für sichtbares Licht dazu ausgelegt ist, das Licht im sichtbaren Spektrum, das von den Beleuchtungszonen (50) der identifizierten freiliegenden Körperteile des Objekts reflektiert wird, in Form einer Bilderserie zu aufzuzeichnen, wobei der Blickwinkel der Vollspektrum-Kamera (30) für sichtbares Licht mit dem Beleuchtungsfeld des programmierbaren Computerprojektors (20) übereinstimmt, und die Auflösung mindestens 1024 Pixel bei einer Bildwiederholfrequenz von mindestens 25-30 Bildern pro Sekunde beträgt, die von einem separaten Software-Thread zur Optimierung und Auswertung unter Verwendung eines programmierbaren Analysators (40) verarbeitet werden.

6. System nach Anspruch 1 oder 3 oder 4 oder 5, **dadurch gekennzeichnet, dass** der vorgenannte programmierbare Analysator (40) ausgestattet ist mit:
- einem Modul zur Erkennung freiliegender Körperteile des Objekts, das dazu ausgelegt ist, Wärmebilder in Echtzeit auf der Grundlage der Erkennung einer Überschreitung eines bestimmten Schwellenwerts der thermischen Emissivität im Vergleich zu bedeckten Körperteilen des Objekts zu analysieren, um einen Satz von Raumkoordinaten zu erhalten, welche die Position und Größe der Konturen der Beleuchtungszonen (50) der identifizierten freiliegenden Körperteile des Objekts angeben, um eine Maske der beleuchteten Bereiche zur späteren Verfolgung während ihrer Bewegung zu erstellen;
- einem Modul zur Erfassung von Vitalparametern, das dazu ausgelegt ist, zyklische Helligkeitsschwankungen der Beleuchtungszonen (50) der identifizierten freiliegenden Körperteile des Objekts zu analysieren und aufeinanderfolgende Wellenwerte der untersuchten Vitalparameter unabhängig für jede der Beleuchtungszonen (50) der identifizierten freiliegenden Körperteile des Objekts zu bestimmen; und
- einem Modul zur Erfassung der Signalqualität der untersuchten Vitalparameter, das dazu ausgelegt ist, die Energie des Spektrums um die Frequenzen der untersuchten Vitalparameter mit der Gesamtenergie des Spektrums zu vergleichen sowie die Stärke und Farbe der einzelnen, von einem programmierbaren Computerprojektor (20) erzeugten Beleuchtungsstrahlen unabhängig voneinander zu steuern.

## Revendications

1. Système d'éclairage sélectif adaptatif destiné à la détection optique des signes vitaux, en particulier conçu pour surveiller ces signes par l'enregistrement de séquences d'images des parties du corps exposées du sujet, comprenant: un détecteur de rayonnement thermique, un éclairage de surface et une caméra à lumière visible, **caractérisé en ce que** le détecteur de rayonnement thermique (10), ainsi que l'éclairage de surface sous la forme d'un projecteur informatique programmable (20) et la caméra à lumière visible (30) à spectre complet, sont reliés entre eux pour former une tête de mesure qui est connectée à un analyseur programmable (40), la tête de mesure étant montée sur un bras support (60) de telle sorte que les surfaces observées et éclairées coïncident et correspondent aux zones d'éclairage (50) identifiées sur les parties du corps exposées du sujet, permettant ainsi de déterminer le signal des signes vitaux par la méthode de la pléthysmographie vidéo (PPG) grâce à l'analyse des variations de luminosité d'une même zone dans des séquences d'images successives en temps réel, dans de nombreuses zones de mesure analysées simultanément et indépendamment les unes des autres.

2. Système selon la revendication 1, **caractérisé en ce que** le détecteur de rayonnement thermique (10), le projecteur informatique programmable (20) et la caméra à lumière visible (30) à spectre complet sont chacun équipés d'objectifs à focale fixe présentant un angle de champ large d'au moins 45 degrés.

3. Système selon la revendication 1 ou 2, **caractérisé en ce que** le détecteur de rayonnement thermique (10) est une caméra thermographique ayant une sensibilité, dans la bande infrarouge, dans la gamme spectrale de 7,5 à 14 µm, avec une précision relative de 50 mK, adaptée à l'enregistrement de l'émissivité thermique d'un objet sous la forme d'une séquence d'images présentant une résolution spatiale d'au moins 2 mm par pixel et une résolution thermique d'au moins 0,5 °C, avec une fréquence de rafraîchissement des images thermiques d'au moins 25 à 30 images thermiques par seconde, gérées par un processus logiciel distinct dédié à l'optimisation et à l'analyse à l'aide d'un analyseur programmable (40).

4. Système selon la revendication 1 ou 2, **caractérisé en ce que** le projecteur informatique programmable (20) est équipé d'un dispositif d'éclairage adapté à l'éclairage sélectif et adaptatif des parties du corps de l'objet qui sont exposées, sur la base d'une émission de lumière sous forme de faisceaux d'éclairage correspondant au nombre de zones d'éclairage (50) des parties du corps de l'objet identifiées comme étant exposées, chacun des faisceaux d'éclairage constituant une source lumineuse indépendante dont l'intensité et la couleur sont contrôlées séparément et varient en fonction des conditions du lieu de mesure et des propriétés optiques de l'objet, et ce, indépendamment pour chaque zone éclairée, gérée par un processus logiciel distinct dédié à l'optimisation et à l'analyse à l'aide d'un analyseur programmable (40).

5. Système selon la revendication 1 ou 2, **caractérisé en ce que** la caméra à lumière visible (30) à gamme spectrale complète est adaptée pour enregistrer la lumière dans la bande visible réfléchie par les zones d'éclairage (50) des parties du corps exposées de l'objet, sous la forme d'une série d'images, l'angle de vision de la caméra à lumière visible (30) à spectre complet coïncidant avec le champ d'éclairage du projecteur informatique programmable (20), et la résolution étant d'au moins 1024 pixels avec une fréquence de rafraîchissement d'au moins 25 à 30 images par seconde, gérées par un processus logiciel distinct dédié à l'optimisation et à l'analyse à l'aide d'un analyseur programmable (40).

6. Système selon la revendication 1 ou 3 ou 4 ou 5, **caractérisé en ce que** ledit analyseur programmable (40) est équipé:
- d'un module de détection des parties du corps de l'objet qui sont exposées, adapté pour analyser des images thermiques en temps réel sur la base de la détection d'un dépassement d'une valeur seuil définie d'émissivité thermique par rapport aux parties du corps de l'objet qui sont couvertes, afin d'obtenir un ensemble de coordonnées spatiales déterminant la position et la taille des contours des zones éclairées (50) des parties du corps exposées identifiées du sujet, afin de définir un masque des zones éclairées en vue de leur suivi ultérieur lors de leur déplacement;
- d'un module de détection signes vitaux, adapté à l'analyse des variations cycliques de la luminosité des zones d'éclairage (50) des parties du corps exposées identifiées de l'objet et de déterminer les valeurs successives des ondes des signes vitaux mesurés indépendamment pour chacune des zones d'éclairage (50) des parties du corps exposées identifiées de l'objet; et
- d'un module de détection de la qualité du signal des signes vitaux mesurés, conçu pour comparer l'énergie spectrale autour de la fréquence de ces signes avec l'énergie spectrale totale, et pour contrôler de manière indépendante l'intensité et la couleur des différents faisceaux lumineux générés par un projecteur informatique programmable (20).
